# EUROPEAN PATENT APPLICATION

(11) **EP 1 023 885 A1**
(43) Date of publication of application: **02.08.2000**
(21) Application number: 99928022.5
(22) Date of filing: 05.07.1999
(51) Int. Cl.: A61F 13/15

(54) **ANAL SANITARY TOWEL**

(30) Priority: 13.07.1998 ES 9801486
(71) Applicant: Gomez Alvarez, Juan José, 33203 Gijon-Asturias (ES); Gomez Alvarez, Eliecer, 33203 Gijon-Asturias (ES); Vallejo Sanchez, Julia Maria, 33203 Gijon-Asturias (ES)
(72) Inventor: GOMEZ MUNIZ, José Antonio, E-33203 Gijon-Asturias (ES)
(74) Representative: Urizar Anasagasti, José Antonio
(86) International application number: ES9900211
(87) International publication number: WO0002508

(57) **Abstract**

It is a pad to be used in anal or perianal area. It is lune-shaped or conical-shaped with an end in wedge formation.

Its structure is comprised by a thin external layer made with cellulose (3) and inner layer of absorbent material (4) which can be impregnated with topic use creams or solutions and the possibility of fixing on underclothes by an adherent base (6). The pad can be used for the absorption of body exudation as well as perspiration produced after or during physical exercises, for persons who have to remain seated during long time periods, has cushion or pad effect in the case of operations or removals and has soothing means to calm irritations produced by piles or the like.

## Description

### Technical Field

This invention just its title aims, it is an anal pad which can be used easily and placed it easily too. It is mainly used to avoid superficial ailments on skin produced by sweat such as rubberings, irritations and spots or pimples. This pad impregnated with a specific medicine it will calm or cure completely irritations produced by pile -and another problems like boils in anal or perianal area.

### State of the Art

Until now it hasn't been known any pad with these features, designed to some determinate applications that we'll describe later. People who suffer from piles, irritations, boils, spots or ingrowing hairs have to use a treatment with gels, cream or ointments on the anal or perianal area. Pants, knickers and underwears of course absorb these products and part of product doesn't work in a correct way. On the other hand there are another cases from people who suffer from urine incontinence, babies with nappy rash or even irritations produced by sweat. These people generally use talcum powder but this powder mixed with sweat produces a scab and this irritates even more the area or use smoothing towels that don't solve the problem neither eliminates it.

In order to solve these problems it has been invented the anal pad that is the object of these invention. This pad will make easy to put creams or another topic use pharmacological product and at the same time people will feel comfortable, clean and without irritations.

### Disclosure of the invention

The anal pad is as its name points a pad which is set aside to be used in the anal or perianal area. It is lune or conical-shaped with a wedge formation at the end. It is easily to use and place. It consists in a thin outer layer made with a non irritant material as cellulose; an inner part made with absorbent material and with the possibility of having an adhesive base in order to be fixed to under clothes. The inner layer absorbs any kind of exudation such as wet produced by flatulencies, sweat produce by sportspeople after or during physical exercise, hard workers, people who have to remain sitting (drivers) or people who are on a wheelchair. All of them generally have any anal or perianal ailments. The anal pad can also be used as tampon in piles operation, or absorbing fluids from intestines as in bleeding piles or diarrhea. The absorbing material is ideal to be impregnated with external use solutions or creams. The anal pad can be used by children or adults because it has different sizes and it collects more or less amount of fluid because it has different thickness.

### Description of Drawings

To complete the descriptions that we are writing and in order to help a better comprehension about the invention features, this descriptive memorandum includes some drawings which are strictly illustrative, no restricted.
Figure 1 shows an inside view of the anal pad.
Figure 2 shows a perspective of the anal pad.
Figure 3 shows how to use the anal pad.

### Preferred embodiment of the invention

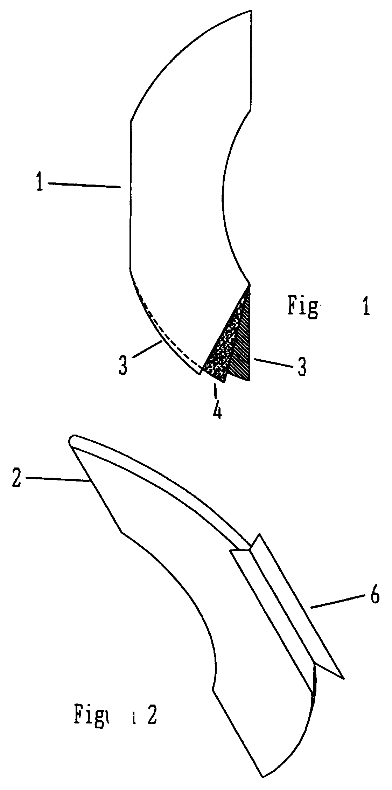

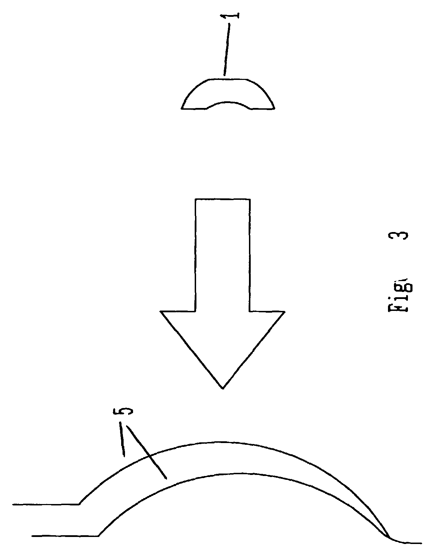

The anal pad (1) is lune-shapped ending with a wedge formation (2) in order to fix it perfectly in anal or perianal area; it has an exterior layer (3) made of a non-irritant by skin contact material such as cellulose and an inner layer (4) made with absorbent material. This anal pad will be used according with figure 3 in direct contact with anal or perianal area (5), it will be fixed on underclothes by adhesive bands if it is big.

It is not considered necessary to long this description. Any expert in the subject will understand the invention and all its advantages. This memorandum will have to be considered in the broad sense of it and no in a limited sense.

Materials, sizes, shape and how to use them could change as long as it doesn't alter on the essential features of the invention which are mentioned in the following paragraph.

## Claims

1. Anal pad used to absorb exudation or body fluids, characterized in that it is lune-shaped ending with a wedge formation, it has an external layer made with non-irritating material when it is in contact to skin and an inner layer with absorbent material.

2. Anal pad, according to the first claim, characterized in that it is provided with an adhesive base to be fixed to underclothes and it doesn't move.

3. Anal pad, according to the first claim, characterized in that can be soaked with different topic use medicine such as creams, solutions or gel.
